# EUROPEAN PATENT SPECIFICATION

(11) **EP 2 119 796 B1**
(45) Date of publication and mention of the grant of the patent: **15.01.2014**
(21) Application number: 08791078.2
(22) Date of filing: 11.07.2008
(51) Int. Cl.: C12Q 1/68

(54) **PROBE FOR DETECTING MUTATION IN JAK2 GENE AND USE THEREOF**
SONDE ZUR ERKENNUNG VON MUTATIONEN DES JAK2-GENS UND ANWENDUNG
SONDE POUR LA DETECTION D'UNE MUTATION DANS LE GENE JAK2 ET SON UTILISATION

(30) Priority: 13.07.2007 JP 2007184842
(43) Date of publication of application: 18.11.2009
(73) Proprietor: ARKRAY, Inc., Kyoto-shi, Kyoto 601-8045 (JP)
(72) Inventor: HIRAI, Mitsuharu, Kyoto-shi Kyoto 601-8045 (JP); MAJIMA, Satoshi, Kyoto-shi Kyoto 601-8045 (JP); MAEKAWA, Taira, Kyoto-shi Kyoto 606-8507 (JP); KIMURA, Shinya, Kyoto-shi Kyoto 606-8507 (JP)
(74) Representative: Jones, Elizabeth Louise
(86) International application number: PCT/JP2008/062548
(87) International publication number: WO 2009/011297

(56) References cited:
- EP-A1- 1 616 952
- WO-A2-02/14555
- JP-A- 2005 287 335
- JP-A- 2005 287 335
- JP-A- 2005 328 707
- JP-A- 2005 328 712
- STEENSMA D P: "JAK2 V617F in Myeloid Disorders: Molecular Diagnostic Techniques and Their Clinical Utility. A Paper from the 2005 William Beaumont Hospital Symposium on Molecular Pathology", JOURNAL OF MOLECULAR DIAGNOSTICS, AMERICAN SOCIETY FOR INVESTIGATIVE PATHOLOGY, BETHESDA, MD, US, vol. 8, no. 4, 1 September 2006 (2006-09-01), pages 397-411, XP008110066, ISSN: 1525-1578, DOI: DOI:10.2353/JMOLDX.2006.060007
- OLSEN RANDALL J ET AL: "Detection of the JAK2(V617F) mutation in myeloproliferative disorders by melting curve analysis using the LightCycler system", ARCHIVES OF PATHOLOGY & LABORATORY MEDICINE, CHICAGO,IL, US, vol. 130, no. 7, 1 July 2006 (2006-07-01), pages 997-1003, XP002445196,
- MCCLURE R ET AL: "Validation of two clinically useful assays for evaluation of JAK2 V617F mutation in chronic myeloproliferative disorders", LEUKEMIA, MACMILLAN PRESS LTD, US, vol. 20, no. 1, 1 January 2006 (2006-01-01), pages 168-171, XP002445193, ISSN: 0887-6924, DOI: DOI:10.1038/SJ.LEU.2404007
- READING N SCOTT ET AL: "Detection of acquired Janus kinase 2 V617F mutation in myeloproliferative disorders by fluorescence melting curve analysis", MOLECULAR DIAGNOSIS AND THERAPY, ADIS INTERNATIONAL LTD, NZ, vol. 10, no. 5, 1 January 2006 (2006-01-01), pages 311-317, XP009144528, ISSN: 1177-1062
- STEENSMA P.D.: 'JAK2 V617F in Myeloid Disorders: Molecular Diagnostic Techniques and Their Clinical Utility' J. MOL. DIAGN. vol. 8, 2006, pages 397 - 411, XP008110066

## Description

### Technical Field

The present invention relates to a probe for detecting a mutation in the JAK2 gene associated with chronic myeloproliferative disorder (CMPD) and the use thereof.

### Background Art

Detection of a point mutation, a so-called single nucleotide polymorphism (SNP), is employed widely as a method of analyzing, at the gene level, for example, the causes of all types of diseases and the individual differences in disease liability (susceptibility to diseases) and in drug action.

Examples of the common methods of detecting a SNP include: (1) a direct sequencing method in which the region where a mutation to be detected occurs is amplified and the base sequence of an amplification product thus obtained is analyzed, (2) a pyrosequencing method, (3) a denaturing HPLC method in which the above-mentioned region is amplified, HPLC is performed in a temperature gradient column, and the presence or absence of any mutation is detected according to the time that is required for elution, (4) an invader method in which using fluorescence that is emitted when a fluorescent probe binds to the region containing a mutation to be detected, a mutation is detected through measurement of fluorescence intensity, and (5) the allele specific primer (ASP) - polymerase chain reaction (PCR) method in which PCR is performed using a primer designed so that any one of bases located in the 3'-end region is a base complementary to a mutation to be detected, and the mutation is judged depending on the presence or absence of amplification.

However, the aforementioned methods (1), (2), and (4) are not very sensitive, specifically their sensitivities are approximately 20%, approximately 5%, and approximately 5%, respectively, and they require a considerable amount of time and labor for their operations. In the aforementioned method (3), the sensitivity is as low as approximately 10%, it only can check the presence or absence of a mutation, and it cannot analyze the site and type of the mutation. Therefore, there is a problem in that it lacks specificity. The aforementioned method (5) is highly sensitive but is less specific, so that it is apt to give a false-positive result, which is a problem. In this context, the lower the numerical value (%) is, the higher the sensitivity.

Because of these problems, recently, melting temperature (Tm) analysis described below is used as a method of detecting a SNP. That is, first, a probe complementary to a region containing a SNP to be detected is used to form a hybrid (double-stranded nucleic acid) between nucleic acid of a sample and the aforementioned probe. Subsequently, this hybridization product is heat-treated, so that dissociation (melting) of the hybrid into a single strand accompanying the temperature rise is detected through measurement of a signal such as absorbance. The Tm value then is determined based on the result of the detection and thereby the presence or absence of the SNP is judged. The higher the homology of the hybridization product, the higher the Tm value, and the lower the horology, the lower the Tm value. Therefore, for example, first, the Tm value (reference value for assessment) is determined beforehand for the hybridization product between nucleic acid containing a variant SNP and a probe complementary thereto. Subsequently, the Tm value (measured value) of the hybridization product between the nucleic acid of the sample and the probe is measured. When the measured value is equal to the reference value for assessment, it is considered as a perfect match, that is, it can be judged that the SNP of the nucleic acid of the sample is a variant. On the other hand, when the measured value is lower than the reference value for assessment, it is considered as a mismatch, that is, it can be judged that the SNP of the nucleic acid of the sample is normal. However, the detection methods using such Tm analysis have a problem in that generally the sensitivity is low.

It has been reported that a variant SNP of the JAK2 gene is found frequently in chronic myeloproliferative disorders (CMPDs) such as polycythemia vera (PV) and essential thrombocythemia (ET) (Nonpatent Documents 1 to 4). The variant SNP is a mutation in which the valine (V) at position 617 of the JAK protein is substituted by phenylalanine (F), and the guanine at position 73 (at position 1849 of mature mRNA (CDS)) in exon 12 of the JAK2 gene is substituted by thymine (JAK2/V617F). It is considered that the detection of the mutation (JAK2/V617F) in the JAK 2 gene that expresses such a protein is useful for diagnoses and treatments of CMPD. However, even in a blood sample of the same patient, the blood sample contains blood cells with a mutated JAK2 gene in which a variant SNP has occurred and with a normal JAK2 gene, a normal SNP. The difference between these genes resides merely in the type of SNP, that is, a single base. Then a phenomenon may occur in which the probe for detecting a variant SNP hybridizes, with perfect match, with the mutant sequence (target sequence) containing the variant SNP and also hybridizes with a normal sequence (non-target sequence) that contains not the variant SNP but a normal SNP even though there is a mismatch in a single base. In such a case, in Tm analysis, when a melting curve that indicates the relationship between signal intensity and temperature is prepared, it is difficult to detect the peak on the higher temperature side that corresponds to the perfectly matched mutant sequence due to the presence of the peak on the lower temperature side that corresponds to the mismatched normal sequence, which is a problem. That is, even when a mutant sequence is present, the presence of a normal sequence makes it difficult to detect the mutant sequence, which causes a decrease in detection sensitivity.

Nonpatent Document 5 reviews various techniques used by research groups and clinical laboratories to detect the genetic mutation underlying JAK2 V617F, including fluorescent dye chemistry sequencing, allele-specific polymerase chain reaction (PCR), real-time PCR, DNA-melting curve analysis, pyrosequencing, and others.

Nonpatent Document 6 discloses a fluorescent probe-based assay for the detection of the JAK2 V617F mutation.

Patent Document 1 describes a method of detecting pancreatic islet amyloid protein mutant gene (IAPP S20G) using melting curve analysis.
[Patent Document 1] EP 1616952 A1.
[Nonpatent Document 1] Tefferi A. Hematology Am Soc Hematol Educ Program. 2006; 240-245.
[Nonpatent Document 2] Baxter EJ, Scott LM, Campbell PJ, et al. Lancet. 2005; 365: 1054-1061.
[Nonpatent Document 3] Kralovics R, Passamonti F, Buser AS, et al. N Engl J Med. 2005; 352: 1779-1790.
[Nonpatent Document 4] Levine RL, Wadleigh M, Cools J, et al. Cancer Cell. 2005; 7: 387-397.
[Nonpatent Document 5] Steensma DP. Journal of Molecular Diagnostics, 8(4), 2006; 397-411.
[Nonpatent Document 6] Reading NS, et al. Mol. Diag. Ther. 10(5), 2006; 311-317.

### Disclosure of Invention

Hence, the present invention is intended to provide a probe for detecting a mutation in the JAK2 gene and the uses thereof. The probe can detect the target mutation with high sensitivity, even when a mutant sequence containing a mutation and a normal sequence containing no mutation coexist, which are different only in a single base from each other.

In order to achieve the above-mentioned object, the probe of the present invention is a probe for detecting a mutation in the JAK2 gene, wherein the probe consists of oligonucleotides (X) having a sequence identical to that of a region extending from a cytosine base (c) at position 84 to be considered as the first base to any one of the 17^{th} to 22^{nd} bases in the direction toward the 5' end in exon 12 of the JAK2 gene consisting of the base sequence of SEQ ID NO: 1, with the cytosine base (c) being the 3' end wherein the cytosine base at the 3' end is labeled with a labeling substance.

A method of detecting a mutation of the present invention is a method of detecting a mutation in the JAK2 gene, wherein the method includes the following steps (A) to (C):
(A) providing a reaction system including a probe according to the present invention and nucleic acid to be tested for detecting the presence or absence of the mutation,
(B) measuring signal values that indicate melting states of a hybridization product between the probe and the nucleic acid to be tested while changing the temperature of the reaction system, and
(C) determining the presence or absence of the mutation in the nucleic acid to be tested, from a change in the signal values accompanying a change in the temperature.

A primer set of the present disclosure is a primer set for amplifying the JAK2 gene by a nucleic acid amplification technique, wherein the primer set contains a pair of primers including a forward primer composed of the following oligonucleotide (F) and a reverse primer composed of the following oligonucleotide (R):
(F): oligonucleotide that is at least one oligonucleotide having a sequence identical to that of a region extending from a cytosine base (c) at position 193 to be considered as the first base to any one of the 24^{th} to 44^{th} bases in the direction toward the 5' end in the base sequence of SEQ ID NO: 2, with the cytosine base (c) being the 3' end, and
(R): oligonucleotide that is at least one oligonucleotide complementary to a region extending from a cytosine base (c) at position 255 to be considered as the first base to any one of the 23^{rd} to 47^{th} bases in the direction toward the 3' end in the base sequence of SEQ ID NO: 2, with a guanine base (g) complementary to the cytosine base (c) at position 255 being the 3'end.

A mutation detection kit of the present invention is a kit that is used for a method of detecting a mutation in the JAK2 gene of the present invention, wherein the kit includes a probe of the present invention.

In the case of using the probe of the present invention, even when a mutant sequence (mutated gene) in which a mutation to be detected, JAK2/V617F, has occurred coexists with a normal sequence (normal gene) in which the mutation has not occurred, the presence or absence of the mutant sequence, i.e. the presence or absence of the mutation to be detected can be detected. As described above, in the Tm analysis, generally, a probe hybridizes to both a normal sequence and a mutant sequence, which are different only in a single base from each other. Accordingly, the signals from both overlap in the melting curve and thereby it is considered to be very difficult to detect the presence or absence of the mutant sequence. On the other hand, although the probes of the present invention hybridize to both a mutant sequence and a normal sequence, the signals from both can be separated sufficiently in the melting curve. Thus, the present invention allows a mutation (JAK2/V617F) in the JAK2 gene to be detected with high sensitivity.

### Brief Description of Drawings

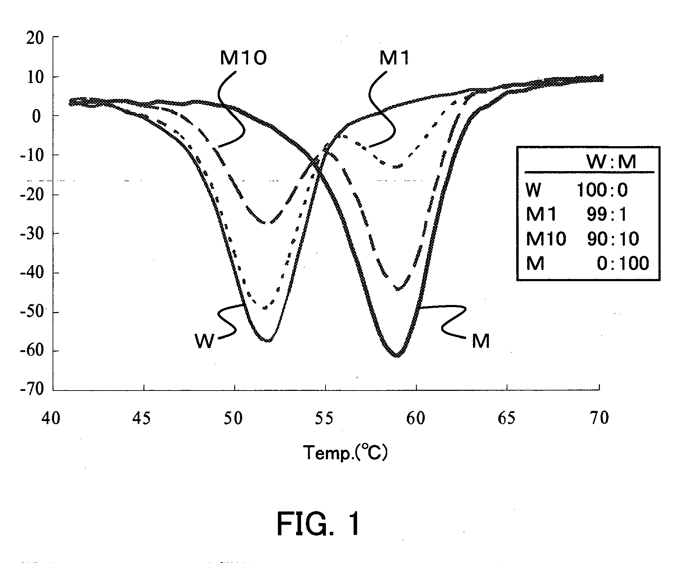

FIG. 1 is a graph showing the results of Tm analysis in Example 1 of the present invention.

### Best Mode for Carrying Out the Invention

Hereinafter, in the present disclosure, the JAK2 gene in which a mutation to be detected has occurred is referred to as a "mutated gene or target gene", and a region of a sequence where a mutation to be detected has occurred, to which a probe can hybridize, or a sequence including the region is referred to as a "mutant sequence or target sequence". Furthermore, the JAK2 gene in which no mutation to be detected has occurred is referred to as a "normal gene or non-target gene", and a region of a sequence where no mutation to be detected has occurred, to which a probe can hybridize, or a sequence including the region is referred to as a "normal sequence or non-target sequence". Moreover, nucleic acid that is used for detection of a mutation also is referred to as "nucleic acid to be tested or target nucleic acid".

The base sequence of SEQ ID NO: 1 is a cDNA sequence of exon 12 in the JAK2 gene, with the base (k) at position 73 being a base to be detected. The base (k) at position 73 is known as a SNP, for example, a normal type g and a mutation type t. The base sequence of SEQ ID NO: 2 is a partial sequence of a full-length sequence (including exons and introns) of the JAK2 gene and a sequence including exon 12 and a region therearound. The full-length sequence (142751 bp) of the JAK2 gene has been registered at NCBI under accession No. NC_000009, for example. Furthermore, mRNA (5097 bp) of the JAK2 gene has been registered at NCBI under accession No.NM_004972, for example. In this registered base sequence, position 2343 is the base site to be detected. In the base sequence that has been registered at NCBI under accession No.NM_004972, a region extending from position 495 to position 3893 is a CDS sequence, position 1849 in the CDS is the base site to be detected.

### <Probe>

As described above, a probe of the present invention is one for detecting a mutation in the JAK2 gene and is characterized by consisting of the oligonucleotides (X) described below. In this context, probes of the present invention may be those containing the aforementioned oligonucleotide on page 4. In the present invention, the oligonucleotide (X) can be any one as long as the 3' end satisfies the following conditions.
(X) Oligonucleotide that is at least one oligonucleotide having a sequence identical to-that of a region extending from a cytosine base (c)-at position 84 to be considered as the first base to any one of the 17^{th} to 22^{nd} bases in the direction toward the 5' end in exon 12 of the JAK2 gene consisting of the base sequence of SEQ ID NO: 1, with the cytosine base (c) being the 3' end.

The probe of the present invention is one for detecting the mutation (g→t) of the base (k) at position 73 in exon 12 of the JAK2 gene that is indicated in SEQ ID NO: 1, i.e. at position 1849 in cDNA (corresponding to mature mRNA) of the JAK2 gene. Among the probes of the present invention, one consisting of the oligonucleotide (X) is complementary to an antisense strand (reverse strand) of the JAK2 gene and can check a mutation in the antisense strand. Furthermore, among the probes of the present disclosure, one consisting of the oligonucleotide (Y) is complementary to a sense strand (forward strand) of the JAK2 gene and can check a mutation in the sense strand.

A probe of the present invention may be designed, for example, to be perfectly matched with a mutant sequence with position 73 being mutated (t) or a normal sequence with position 73 being normal (g) in the JAK2 gene.

In the probes of the present invention, examples of the oligonucleotide (X) include those indicated by SEQ ID NOs: 3 to 8. In the following sequences, k may be either g or t, and it is preferably "t" when the probe is designed to be matched perfectly with a mutant sequence while it is preferably "g" when the probe is designed to be perfectly matched with a normal sequence. Particularly, a preferable probe is one consisting of oligonucleotide expressed by SEQ ID NO: 5, and a more preferable probe is one consisting of oligonucleotide expressed by SEQ ID NO: 5, with the base k being thymine.
5'-tatgtktctgtggagac-3' (SEQ ID NO: 3)
5'-gtatgtktctgtggagac-3' (SEQ ID NO: 4)
5'-agtatgtktctgtggagac-3' (SEQ ID NO: 5)
5'-gagtatgtktctgtggagac-3' (SEQ ID NO: 6)
- 5'-ggagtatgtktctgtggagac-3' (SEQ ID NO: 7)
5'-tggagtatgtktctgtggagac-3' (SEQ ID NO: 8)

These oligonucleotides each have a sequence that hybridize to the sequence complimentary to that of a region extending from position 62, 63, 64, 65, 66, or 67 to position 84 (position 73 is g or t) in the base sequence of SEQ ID NO: 1.

Each probe of the present invention is a probe labeled with a labeling substance. The labeling substance is not limited and is, for example, a fluorescent dye (fluorophore). A specific example of the labeled probes is preferably, for example, a probe that has been labeled with the fluorescent dye, exhibits fluorescence independently, and allows fluorescence to be reduced (for example, quenched) after hybridization. Generally, such a phenomenon is referred to as a fluorescence quenching phenomenon.
Among probes that utilize the fluorescence quenching phenomenon, one referred to generally as a "guanine quenching probe" is preferable. Such a probe is known as so-called QProbe (registered trademark). The guanine quenching probe is, for example, a probe that is designed so that the base at the 3' end or the 5' end of oligonucleotide is cytosine, with the end being labeled with a fluorescent dye whose emission decreases as the cytosine base located at the end approaches a guanine base complementary thereto. In each probe of the present invention, for instance, a fluorescent dye that exhibits the fluorescence quenching phenomenon may be bonded to cytosine at the 3' end of the oligonucleotide, or may be bonded to cytosine at the 5' end of the oligonucleotide when the 5' end is designed to be cytosine.

The above-mentioned fluorescent dye is not particularly limited. Examples thereof include fluorescein, phosphor, rhodamine, and polymethine dye derivative. Examples of the commercially available fluorescent dye include BODIPY FL (registered trade name, manufactured by Molecular Probe Inc.), FluorePrime (trade name, manufactured by Amersham Pharmacia), Fluoredite (trade name, manufactured by Millipore Corporation), FAM (manufactured by ABI), Cy3 and Cy5 (manufactured by Amersham Pharmacia), and TAMRA (manufactured by Molecular Probe Inc.). The conditions for detecting a probe are not particularly limited and can be determined suitably according to the fluorescent dye to be used. For example, it can be detected at a detection wavelength of 450 to 480 nm in the case of Pacific Blue, at a detection wavelength of 585 to 700 nm in the case of TAMRA, and at a detection wavelength of 515 to 555 nm in the case of BODIPY FL. The uses of such probes make it possible to check hybridization and dissociation easily by a change in signal.

A probe of the present invention may include, for example, a phosphate group added to the 3' end thereof. As described later, nucleic acid to be tested (target nucleic acid) for detecting the presence or absence of a mutation can be prepared by a nucleic acid amplification technique such as PCR. In this case, a probe of the present invention is allowed to coexist in the reaction system in a nucleic acid amplification reaction. In such a case, when a phosphate group has been added to the 3' end of the probe, this sufficiently can prevent the probe itself from elongating through the nucleic acid amplification reaction. Furthermore, the same effect also can be obtained through addition of a labeling substance described above to the 3' end.

As described above, a probe of the present invention can be used for detecting a mutation in the JAK2 gene. The method of detecting the mutation is not particularly limited and may be any method as long as it utilizes hybridization between a nucleic acid to be tested and the aforementioned probe. A method of detecting a mutation by using Tm analysis is described below as an example of a method in which a probe of the present invention is used.

### <Mutation Detection Method>

As described above, a mutation detection method of the present invention is a method of detecting a mutation in the JAK2 gene and is characterized by including the following steps (A) to (C):
(A) providing a reaction system including a probe of the present invention and nucleic acid to be tested for detecting the presence or absence of the mutation,
(B) measuring signal values that indicate melting states of a hybridization product between the probe and the nucleic acid to be tested while changing the temperature of the reaction system, and
(C) determining the presence or absence of the mutation in the nucleic acid to be tested, from a change in the signal values accompanying a change in the temperature.

The mutation detection method of the present invention makes it possible to detect a mutation (g→t) of the base (k) at position 73 in exon 12 of the JAK2 gene that is indicated in SEQ ID NO: 1. The mutation detection method of the present invention allows detection to be performed with higher sensitivity as compared to, for example, a conventional direct sequencing method or pyrosequencing method.

The nucleic acid to be tested in the present invention may be, for example, a single strand or a double strand. In the case of the double strand, it is preferable that a step of dissociating the double strand into a single strand by heating be included in order to form a hybrid by hybridizing nucleic acid to be tested with a probe, for example.

The type of the nucleic acid to be tested is not particularly limited. Examples thereof include DNAs and RNAs such as total RNA and mRNA. Furthermore, examples of the aforementioned nucleic acid to be tested include nucleic acid contained in a sample such as a biological sample. The nucleic acid contained in the sample may be, for example, nucleic acid that is contained originally in a biological sample but may be an amplification product obtained through amplification by the nucleic acid amplification technique using, as a template, nucleic acid contained in the biological sample, since it can improve the mutation detection accuracy. Specific examples thereof include an amplification product obtained through amplification by the nucleic acid amplification technique using, as a template, DNA contained originally in the biological sample and an amplification product obtained through amplification by the nucleic acid amplification technique using, as a template, cDNA produced by reverse transcription PCR (RT-PCR) from RNA that is contained originally in the biological sample. These amplification products each may be used as nucleic acid to be tested in the present invention. The length of each amplification product is not particularly limited and is, for example, 50 to 1000 bases and preferably 80 to 200 bases.

The aforementioned biological sample is not particularly limited and examples thereof include a blood cell sample such as a white blood cell, and a whole blood sample. In the present invention, for example, the sampling method and the method of preparing nucleic acid such as DNA or RNA are not limited, and conventionally known methods can be employed.

As described above, when the nucleic acid to be tested is an amplification product obtained through amplification of template nucleic acid by the nucleic acid amplification technique, the aforementioned step (A) further may include a step of amplifying template nucleic acid using a primer to obtain nucleic acid to be tested.

The above-mentioned primer is not particularly limited as long as a region to which a probe of the present invention can hybridize in the JAK2 gene is amplified. The region to be amplified may be, for example, only a region to which the probe hybridizes or a region including the hybridization region. The primer may be, for example, either a forward primer or a reverse primer, but it is preferable that both of them be used as a pair. The primer can be any one of those described later but is not limited thereto.

In the present invention, the ratio of the probe of the present invention to be added is not limited. However, from the view point of obtaining detection signals sufficiently, the molar ratio of the probe of the present invention to nucleic acid to be tested is preferably 1:1 or lower and more preferably 0.1:1 or lower. In this case, the nucleic acid to be tested may be, for example, the total of mutant sequence (target sequence) in which a mutation to be detected has occurred and normal sequence (non-target sequence) in which the mutation has not occurred, or the total of an amplification product containing the mutant sequence and an amplification product containing the normal sequence. Generally, the ratio of the mutant sequence in a sample containing nucleic acid to be tested is unknown. However, in terms of the results, the ratio (molar ratio) of the probe to be added to a mutant sequence or an amplification product containing it is preferably 10:1 or lower, more preferably 5:1 or lower, and further preferably 3:1 or lower. Moreover, the lower limit thereof is not particularly limited and is, for example, at least 0.001:1, preferably at least 0.01:1, and more preferably at least 0.1:1.

The ratio of the probe of the present invention to be added to the nucleic acid to be tested may be, for example, a molar ratio thereof to double-stranded nucleic acid or a molar ratio thereof to single-stranded nucleic acid.

The Tm value is described below. For instance, when a solution containing double-stranded DNA is heated, the absorbance at 260 nm increases. This is because heating releases the hydrogen bond between both strands in the double-stranded DNA to dissociate it into single-stranded DNA (melting of DNA). When all double-stranded DNAs are dissociated into single-stranded DNAs, the absorbance thereof indicates approximately 1.5 times that obtained at the start of heating (i.e. absorbance of only double-stranded DNAs), which makes it possible to judge that melting is completed thereby. Based on this phenomenon, the melting temperature Tm generally is defined as a temperature at which the absorbance has reached 50% of the total increase in absorbance.

In the present invention, the measurement of a change in the signal accompanying a change in the temperature for determination of the Tm value can be carried out by measuring absorbance at 260 nm based on the principle as described above but is preferably a measurement of the signal of a label added to the probe of the present invention. Accordingly, it is preferable that the aforementioned labeled probe be used as the probe of the present invention. The labeled probe can be, for example, a labeled probe that exhibits a signal independently but does not exhibit a signal after hybridization or a labeled probe that does not exhibit a signal independently but exhibits a signal after hybridization. The former probe does not exhibit a signal after forming a hybrid (double strand) with a target sequence but exhibits a signal when the probe is released by heating. On the other hand, the latter probe exhibits a signal after forming a hybrid (double strand) with a target sequence but the signal is reduced (quenched) when the probe is released by heating. Accordingly, when the signal exhibited by the label is detected under a condition specific to the signal (for example, absorbance), the progress of melting and the Tm value can be determined as in the case of measuring absorbance at 260 nm. The labeling substance of the labeled probe is, for example, as described above.

Next, the mutation detection method of the present invention is described using an example in which a labeled probe that has been labeled with a fluorescent dye is used as a probe of the present invention. The mutation detection method of the present invention is characterized by the use of a probe of the present invention itself and is not limited by other steps or conditions by any means.

First, genomic DNA is isolated from whole blood. Isolation of the genomic DNA from whole blood can be carried out by a conventionally known method. For example, a commercially available genomic DNA isolation kit (trade name: GFX Genomic Blood DNA Purification kit; manufactured by GE Healthcare Bioscience) can be used.

Next, a labeled probe is added to a sample containing the genomic DNA thus isolated. Preferably, the labeled probe is, for example, QProbe. This QProbe generally is a probe in which a cytosine base located at the end thereof is labeled with a fluorescent dye. When it hybridizes to a target sequence, the fluorescent dye and a guanine base of the target sequence interact with each other and as a result, the fluorescence decreases (or quenches).

The timing for adding the probe is not particularly limited. For instance, the probe may be added to the reaction system for the amplification reaction to be described later, before, during, or after the amplification reaction. Particularly, it is preferable that the probe be added before the amplification reaction since this allows the amplification reaction and the aforementioned step (B) to be performed successively. When the probe is added before the nucleic acid amplification reaction in that manner, it is preferable that, for example, a fluorescent dye or a phosphate group be added to the 3' end thereof, as described above.

The aforementioned probe may be added to a liquid sample containing isolated genomic DNA or may be mixed with genomic DNA in a solvent. The solvent is not particularly limited. Examples thereof include conventionally known solvents such as buffer solutions such as Tris-HCl, solvents containing, for example, KCl, MgCl₂, MgSO₄, or glycerol, and PCR reaction solutions.

Subsequently, with isolated genomic DNA used as a template, a sequence containing a base site to be detected is amplified by a nucleic acid amplification technique such as PCR. The nucleic acid amplification technique is not limited. Examples thereof include the polymerase chain reaction (PCR) method, a nucleic acid sequence based amplification (NASBA) method, a transcription-mediated amplification (TMA) method, and a strand displacement amplification (SDA) method. Particularly, the PCR method is preferable. The present invention is described below using the PCR method as an example but is not limited thereto. The conditions for PCR are not particularly limited and it can be carried out by the conventionally known method.

The sequence of a primer for PCR is not particularly limited as long as it can amplify a sequence containing the base site to be detected. As described above, it can be designed suitably by a conventionally known method according to the target sequence. The length of the primer is not particularly limited and can be set at a general length (for example, 10- to 30-mers).

The primer set that is used for the detection method of the present invention can be, for example, a primer set of the present invention to be described later. The intended use of the primer set of the present invention is not limited to the detection method of the present invention. Furthermore, the primer set that is used for the detection method of the present invention also is not limited to the primer set of the present invention.

Next, the resultant PCR amplification product is dissociated and the single-stranded DNA obtained thereby is hybridized with the labeled probe. This can be carried out by, for example, changing the temperature of the reaction solution.

The heating temperature employed in the dissociation step is not particularly limited as long as it allows the amplification product to be dissociated. It is, for example, 85 to 95°C. The heating time also is not particularly limited and generally is 1 second to 10 minutes and preferably 1 second to 5 minutes.

The dissociated single-stranded DNA can be hybridized with the labeled probe by, for example, decreasing the heating temperature employed in the dissociation step after the dissociation step. The temperature condition is, for example, 40 to 50°C.

The volume and concentration of each composition in the reaction system (reaction solution) in the hybridization step are not particularly limited. Specifically, in the reaction system, the concentration of DNA is, for example, 0.01 to 1 µmol/L and preferably 0.1 to 0.5 µmol/L, and the concentration of the labeled probe is preferably in a range that satisfies, for example, the ratio thereof to be added to the DNA, for instance, 0.001 to 10 µmol/L and preferably 0.001 to 1 µmol/L.

The temperature of the reaction solution further is changed, and thereby signal values that indicate the melting states of a hybridization product between the amplification product and the labeled probe are measured. Specifically, for example, the reaction solution (the hybridization product between the single-stranded DNA and the labeled probe) is heated, and thereby the change in the signal values accompanying the temperature rise is measured. As described above, when a probe (guanine quenching probe), in which the base C at the end has been labeled, is used, fluorescence decreases (or quenches) in the state where the probe has hybridized with the single-stranded DNA, while fluorescence is emitted in the state where the probe has been dissociated. Accordingly, for example, the hybridization product in which the fluorescence has decreased (or quenched) is heated gradually and thereby the increase in fluorescence intensity accompanying the temperature rise may be measured.

The temperature range over which the change in fluorescence intensity is measured is not particularly limited. For example, the start temperature is from room temperature to 85°C, preferably 25 to 70°C, while the end temperature is, for example, 40 to 105°C. Furthermore, the rate of temperature rise is not particularly limited and is, for example, 0.1 to 20°C/sec and preferably 0.3 to 5°C/sec.

Next, the change in the signal is analyzed and thereby the Tm value is determined. Specifically, the amount of change in the fluorescence intensity per unit time at each temperature is calculated from the fluorescence intensity obtained. When the amount of change is expressed as (-d fluorescence intensity increase/dt), for example, the temperature at which the lowest-value-is-obtained-is determined as the Tm value. It also is possible that when the amount of change is expressed as (d fluorescence intensity increase/t), for example, the point at which the highest value is obtained is determined as the Tm value. Conversely, the amount of decrease in the fluorescence intensity may be measured when the labeled probe used is not a quenching probe but a probe that does not exhibit a signal independently but exhibits a signal after hybridization.

The Tm values can be calculated with, for example, the conventionally known MELTCALC software (http://www.meltcalc.com/), or it also can be determined by the nearest neighbor method.

From such a Tm value, the genotype (for example, mutation type or normal type) at a target base site is determined. In the Tm analysis, the case of a perfectly complementary hybrid (perfect match) results in a higher Tm value indicating dissociation than that obtained in the case of a hybrid including a different single base (mismatch). Accordingly, when, with respect to the probe, the Tm value obtained in the case of a perfectly complementary hybrid and the Tm value obtained in the case of a hybrid including a different single base are determined beforehand, the genotype at the target base site can be determined. For example, in the case where the base at the target base site is assumed to be of a mutation type, when using a probe complementary to the target sequence containing the base of the mutation type, the target base can be judged as a mutation type if the Tm value of the resultant hybrid is equal to the Tm value of a perfectly complementary hybrid. Furthermore, the target base can be judged as a normal type if the Tm value of the resultant hybrid is equal to the Tm value of the hybrid including a different single base (i.e. a lower value than the Tm value of the perfectly complementary hybrid).

In the present invention, for example, a change in the signal during hybridization may be measured instead of the method in which the temperature of a reaction solution containing the probe is increased (i.e. a hybridization product is heated) and a change in the signal accompanying the temperature rise is measured as described above. In other words, when the temperature of the reaction solution containing the aforementioned probe is decreased and thereby a hybridization product is formed, the change in the signal accompanying the temperature decrease may be measured.

Specifically, when using a labeled probe that exhibits a signal independently but does not exhibit a signal after hybridization (for example, a guanine quenching probe), the labeled probe emits fluorescence in the state where single-stranded DNA and the probe are dissociated, but the fluorescence decreases (or quenches) when a hybrid is formed through a temperature decrease. Accordingly, for example, while the temperature of the reaction solution is decreased gradually, the decrease in fluorescence intensity accompanying the temperature decrease may be measured. On the other hand, when using a labeled probe that does not exhibit a signal independently but exhibits a signal after hybridization, the labeled probe does not emit fluorescence in the state where single-stranded DNA and the probe are dissociated, but the fluorescence is emitted when a hybrid is formed through the temperature decrease. Accordingly, for example, while the temperature of the reaction solution is decreased gradually, the increase in fluorescence intensity accompanying the temperature decrease may be measured.

### <Primer Set>

A primer set of the present disclosure is one for amplifying the JAK2 gene by a nucleic acid amplification technique and is characterized by containing a pair of primers including a forward primer composed of the following oligonucleotide (F) and a reverse primer composed of the following oligonucleotide (R):
(F): oligonucleotide that is at least one oligonucleotide having a sequence identical to that of a region extending from the cytosine base (c) at position 193 to be considered as the first base to any one of the 24^{th} to 44^{th} bases in the direction toward the 5'end in the base sequence of SEQ ID NO: 2; with the cytosine base (c) being the 3' end, and
(R): oligonucleotide that is at least one oligonucleotide complementary to a region extending from the cytosine base (c) at position 255 to be considered as the first base to any one of the 23^{rd} to 47^{th} bases in the direction toward the 3' end in the base sequence of SEQ ID NO: 2, with a guanine base (g) complementary to the cytosine base (c) position 255 being the 3' end.

According to the present disclosure, each primer may be any primer as long as the base located at the 3' end that serves to determine the starting point for amplification that is performed using DNA polymerase satisfies the aforementioned conditions.

As described above, since the forward primer and reverse primer each can be any primer as long as the base located at the 3' end is fixed, the length itself of each primer is not particularly limited and can be adjusted suitably to a common length. Each length of the primers is, for example, in the range of 13- to 50-mers, preferably 14- to 45-mers, and more preferably 15- to 40-mers. Specifically, it is preferable that the forward primer be at least one oligonucleotide having a sequence identical to that of a region extending from the cytosine base (C) at position 193 to be considered as the first base to any one of the 24^{th} to 47^{th} bases (preferably the 25^{th} to 35^{th} bases and more preferably the 27^{th} to 32^{nd} bases) in the direction toward the 5' end in the base sequence of SEQ ID NO: 2. Furthermore, it is preferable that the reverse primer be at least one oligonucleotide complementary to a region extending from the cytosine base (C) at position 255 to be considered as the first base to any one of the 23^{rd} to 47^{th} bases (preferably the 25^{th} to 38^{th} bases and more preferably the 26^{th} to 31^{st} bases) in the direction toward the 3' end in the base sequence of SEQ ID NO: 2. Since each 3' end of the forward primer and the reverse primer is fixed, the region to be elongated from the primer is, for example, a region between position 194 and position 254 in SEQ ID NO: 2. However, the length of the whole resultant amplification product varies according to the length of the primer to be used.

Furthermore, it is not necessary for the reverse primer and the forward primer to be oligonucleotides perfectly complementary to the base sequence indicated in SEQ ID NO: 2 and to that of the strand complementary to the aforementioned base sequence, respectively. In other words, the part excluding the base located at the 3' end in each primer may be different in one to five bases from that of a perfectly complementary oligonucleotide.

Specific examples of the forward primer and reverse primer are indicated below but the present invention is not limited thereto. Particularly, a preferable forward primer is SEQ ID NO: 25 and a preferable reverse primer is SEQ ID NO: 51.

**[Table 1]**

| <Forward Primer> | SEQ ID NO |
|---|---|
| 5' -ttagtctttctttgaagcagcaagtatgatgagcaagctttctc-3' | 9 |
| 5' -tagtctttctttgaagcagcaagtatgatgagcaagctttctc-3' | 10 |
| 5' -agtctttctttgaagcagcaagtatgatgagcaagctttctc-3' | 11 |
| 5' -gtctttctttgaagcagcaagtatgatgagcaagctttctc-3' | 12 |
| 5' -tctttctttgaagcagcaagtatgatgagcaagctttctc-3' | 13 |
| 5' -ctttctttgaagcagcaagtatgatgagcaagctttctc-3' | 14 |
| 5' -tttctttgaagcagcaagtatgatgagcaagctttctc-3' | 15 |
| 5' -ttctttgaagcagcaagtatgatgagcaagctttctc-3' | 16 |
| 5' -tctttgaagcagcaagtatgatgagcaagctttctc-3' | 17 |
| 5' -ctttgaagcagcaagtatgatgagcaagctttctc-3' | 18 |
| 5' -tttgaagcagcaagtatgatgagcaagctttctc-3' | 19 |
| 5' -ttgaagcagcaagtatgatgagcaagctttctc-3' | 20 |
| 5' -tgaagcagcaagtatgatgagcaagctttctc-3' | 21 |
| 5' -gaagcagcaagtatgatgagcaagctttctc-3' | 22 |
| 5' -aagcagcaagtatgatgagcaagctttctc-3' | 23 |
| 5' -agcagcaagtatgatgagcaagctttctc-3' | 24 |
| 5' -gcagcaagtatgatgagcaagctttctc-3' | 25 |
| 5' -cagcaagtatgatgagcaagctttctc-3' | 26 |
| 5' -agcaagtatgatgagcaagctttctc-3' | 27 |
| 5' -gcaagtatgatgagcaagctttctc-3' | 28 |
| 5' -caagtatgatgagcaagctttctc-3' | 29 |
| 5' -aagtatgatgagcaagctttctc-3' | 30 |

**[Table2]**

| <Reverse Primer> | SEQ ID NO |
|---|---|
| 5' -ctatataaacaaaaacagatgctctgagaaaggcattagaaagcctg-3' | 31 |
| 5' -tatataaacaaaaacagatgctctgagaaaggcattagaaagcctg-3' | 32 |
| 5' -atataaacaaaaacagatgctctgagaaaggcattagaaagcctg-3' | 33 |
| 5' -tataaacaaaaacagatgctctgagaaaggcattagaaagcctg-3' | 34 |
| 5' -ataaacaaaaacagatgctctgagaaaggcattagaaagcctg-3' | 35 |
| 5' -taaacaaaaacagatgctctgagaaaggcattagaaagcctg-3' | 36 |
| 5' -aaacaaaaacagatgctctgagaaaggcattagaaagcctg-3' | 37 |
| 5' -aacaaaaacagatgctctgagaaaggcattagaaagcctg-3' | 38 |
| 5' -acaaaaacagatgctctgagaaaggcattagaaagcctg-3' | 39 |
| 5' -caaaaacagatgctctgagaaaggcattagaaagcctg-3' | 40 |
| 5' -aaaaacagatgctctgagaaaggcattagaaagcctg-3' | 41 |
| 5' -aaaacagatgctctgagaaaggcattagaaagcctg-3' | 42 |
| 5' -aaacagatgctctgagaaaggcattagaaagcctg-3' | 43 |
| 5' -aacagatgctctgagaaaggcattagaaagcctg-3' | 44 |
| 5' -acagatgctctgagaaaggcattagaaagcctg-3' | 45 |
| 5' -cagatgctctgagaaaggcattagaaagcctg-3' | 46 |
| 5' -agatgctctgagaaaggcattagaaagcctg-3' | 47 |
| 5' -gatgctctgagaaaggcattagaaagcctg-3' | 48 |
| 5' -atgctctgagaaaggcattagaaagcctg-3' | 49 |
| 5 '-tgctctgagaaaggcattagaaagcctg-3' | 50 |
| 5' -gctctgagaaaggcattagaaagcctg-3' | 51 |
| 5' -ctctgagaaaggcattagaaagcctg-3' | 52 |
| 5' -tctgagaaaggcattagaaagcctg-3' | 53 |
| 5' -ctgagaaaggcattagaaagcctg-3' | 54 |
| 5' -tgagaaaggcattagaaagcctg-3' | 55 |

The molar ratio between the forward primer and the reverse primer in each primer set is not particularly limited. Furthermore, the molar ratio between them at the time of the nucleic acid amplification reaction also is not particularly limited but, for example, the molar ratio (F:R) between the forward primer (F) and the reverse primer (R) is preferably 1:0.01 to 100, more preferably 1:0.1 to 10, and particularly preferably 1:0.5 to 2.

### <Mutation Detection Kit>

A mutation detection kit of the present invention is one that is used for a method of detecting a mutation in the JAK2 gene of the present invention and is characterized by including a probe of the present invention. The present invention is characterized by using a probe of the present invention described above.

Furthermore, the method for use of the present invention may be carried out according to the mutation detection method of the present invention.

The mutation detection kit of the present invention further may include one of a forward primer and a reverse primer of a primer set of the present invention or may include both of them. Moreover, the mutation detection kit of the present invention further may include, for example, a reagent that is necessary for amplifying nucleic acid and instructions for use.

Next, examples of the present invention are described. However, the present invention is not limited by the following examples but is defined by the claims which are appended to the end of this description.

### [Example 1]

### Detection of Point Mutation (g→t) of Base at Position 73 in Exon 12 of JAK2 Gene

Tm analysis was performed with respect to a point mutation (g→t) of the base at position 79 in exon 12 of the JAK2 gene indicated in SEQ ID NO: 1 using a probe of the present invention.

### <Sample>

HEL, a JAK2/V617F-positive leukemia cell strain, and MYL, a JAK2 wild-type leukemia strain, were used. The culture media of the respective cells were mixed together so as to have the following ratios of cell counts thereof. Thereafter, 10 µl of each mixed culture medium was treated at 95°C for five minutes and a sample thus obtained then was used as a PCR sample.

**[Table 3]**

| HEL (mt DNA) | : | MYL (wild DNA) |
|---|---|---|
| 0 | : | 100 |
| 0 pc | : | 1 × 10⁶ pcs |
| 1 | : | 99 |
| 1 × 10⁴ pcs | : | 99 × 10⁴ pcs |
| 10 | : | 90 |
| 1 × 10⁵ pcs | : | 9 × 10⁵ pcs |
| 100 | : | 0 |
| 1 × 10⁶ pcs | : | 0 pc |

### <Probe and Primer Set>

Detection Probe (SEQ ID NO: 5)
   5'-agtatgtTtctgtggagac-(TAMRA)-3'
Forward Primer (SEQ ID NO: 25)
   5'-gcagcaagtatgatgagcaagctttctc-3'
Reverse Primer (SEQ ID NO: 51)
   5'-gctctgagaaaggcattagaaagcctg-3'

### <Tm analysis>

A mixture of 20 µl of the following P-mix and 25 µl of the following E-mix was added to 5 µl of the PCR sample and thereby a PCR reaction was performed. In the PCR reaction, using a thermal cycler, after it was treated at 95°C for 60 seconds, one cycle of treatment at 95°C for five seconds and 58°C for 30 seconds was repeated for 35 to 50 cycles, and further it was treated at 95°C for 1 second and at 40°C for 60 seconds. Subsequently, the PCR reaction solution was heated from 40°C to 70°C at a rate of temperature rise of 1°C/3 seconds, and the change in fluorescence intensity over time was measured (at a wavelength of 585 to 700 nm).

**[Table 4]**

| <P-mix> | | <E-mix> | |
|---|---|---|---|
| Distilled water | 18.75 | Distilled water | 8.5 |
| 100 µmol/L Forward Primer | 0.25 | 2.5 mmol/L dNTP | 4 |
| 100 µmol/L Reverse Primer | 0.5 | 10×Gene Taq buffer* | 5 |
| 5 µmol/L Detection Probe | 0.5 | 40% Glycerol | 6.25 |
| Total | 20 µL | 20% BSA | 1 |
| | | 5 U/µl Gene Tag FP* | 0.25 |
| | | Total | 25 µL |

| | | | |
|---|---|---|---|
| * Trade name, Gene Taq FP manufactured by Nippon Gene Co., Ltd. | | | |

The results are shown in FIG. 1. FIG. 1 shows a Tm analysis graph that indicates changes in fluorescence intensity accompanying temperature rise. In FIG. 1, W indicates the results of wild (normal type) DNA (100%), M those of mt (mutation type) DNA (100%), M1 those of a mixture of mt DNA and wild DNA mixed at a ratio of 1 : 99, and M10 those of a mixture of mt DNA and wild DNA mixed at a ratio of 10 : 90. As indicated with W in FIG. 1, the wild DNA (100%) had a peak at 52°C, while the mt DNA (100%) had a peak at 58°C as indicated with M. With these peak temperatures (Tm values) used as evaluation references, the peaks of the mixtures of wild DNA and mt DNA were evaluated. Consequently, as indicated with M1 and M10 in FIG. 1, as a result of the use of the above-mentioned detection probe, the peaks of wild DNA and mt DNA were detected at equivalent temperatures to those of the aforementioned evaluation references, respectively. As a result, it was proved that the probe of the present invention allowed sufficiently high detection sensitivity with respect to mt DNA to be obtained even when the mt DNA and the wild DNA coexisted. Furthermore, in the case of a general direct sequencing or pyrosequencing method, the detection sensitivity was approximately 5 to 20%, that is, it was difficult to detect mt DNA unless 5 to 20% thereof existed, but the Tm analysis using the detection probe made it possible to detect it well even when mt DNA was approximately 1%. Thus, it was proved that the probe of the present invention had a very high sensitivity.

Furthermore, blood was collected from patients suffering from chronic myeloproliferative disorder (CMPD), and genomic DNA was extracted from each sample. Thereafter, Tm analysis was carried out in the same manner as described above with respect to the mutation in the JAK2 gene. As a result, with respect to the plurality of patients, JAK2-V617F was detected in all the polycythemia vera cases (PV case : 10 cases) and essential thrombocythemia cases (ET case : 4 cases). With respect to the ET cases, it was under detection limit in the direct sequencing method. Accordingly, it was proved that the method of this example had a very high detection sensitivity and was very useful.

### Industrial Applicability

As described above, even when the JAK2 gene (mutated gene) in which a mutation to be detected has occurred coexists with the JAK2 gene (normal gene) in which no mutation has occurred, each probe of the present invention can detect the mutated gene including the mutation to be detected. For instance, in the Tm analysis, since a conventional probe hybridizes to both a mutated gene and a normal gene, which are different only in a single base from each other as described above, the signals from both overlap in the melting curve and thereby it is very difficult to detect the presence of the mutated gene. On the other hand, although the probes of the present invention each hybridize to both a mutated gene and a normal gene, the signals from both can be separated in the melting curve. Furthermore, the method using a probe of the present invention has higher detection sensitivity than that obtained in known methods such as the direct sequencing method and the pyrosequencing method. Thus, the present invention makes it possible to detect a mutated gene with high sensitivity, for example, even by the Tm analysis.

## Claims

1. A probe for detecting a mutation in the JAK2 gene,
wherein the probe consists of oligonucleotide (X) having a sequence identical to that of a region extending from a cytosine base (c) at position 84 to be considered as the first base to any one of the 17^{th} to 22^{nd} bases in a direction toward the 5' end in exon 12 of the JAK2 gene consisting of the base sequence of SEQ ID NO: 1, with the cytosine base (c) being the 3' end, wherein the cytosine base at the 3' end is labeled with a labeling substance.

2. The probe according to claim 1, wherein oligonucleotide (X) is an oligonucleotide consisting of a base sequence selected from the group consisting of SEQ ID NOs: 3 to 8, or
wherein a base (k) in the base sequence of SEQ ID NOs: 3 to 8 is thymine (t), or
wherein the probe is one for detecting a mutation (g→t) of a base at position 73 in the base sequence of SEQ ID NO: 1.

3. The probe according to claim 1, wherein the labeled probe is one that exhibits a signal independently but does not exhibit a signal after hybridization or one that does not exhibit a signal independently but exhibits a signal after hybridization, or
wherein the labeled probe is a probe labeled with a fluorescent dye and is a probe that exhibits fluorescence independently and allows fluorescence to decrease after hybridization.

4. The probe according to claim 1, wherein the probe is a probe for melting temperature (Tm) analysis.

5. A method of detecting a mutation in the JAK2 gene,
wherein the method comprises the following processes (A) to (C):
(A) providing a reaction system including a probe according to claim 1 and nucleic acid to be tested for detecting the presence or absence of the mutation,
(B) measuring signal values that indicate melting states of a hybridization product between the probe and the nucleic acid to be tested while changing the temperature of the reaction system, and
(C) determining the presence or absence of the mutation in the nucleic acid to be tested, from a change in the signal values accompanying a change in the temperature.

6. The method according to claim 5, wherein the mutation in the JAK2 gene is a mutation (g→t) of a base at position 73 in exon 12 of the JAK2 gene indicated in SEQ ID NO: 1.

7. The method according to claim 5, wherein the nucleic acid to be tested that is used in the process (A) is an amplification product that has been amplified from template nucleic acid by a nucleic acid amplification technique.

8. The method according to claim 7, wherein the process (A) further comprises a process of amplifying nucleic acid to be tested from template nucleic acid using a primer set.

9. The method according to claim 8, wherein the primer set comprises a pair of primers including a forward primer composed of the following oligonucleotide (F) and a reverse primer composed of the following oligonucleotide (R):
(F): oligonucleotide having a sequence identical to that of a region extending from a cytosine base (c) at position 193 to be considered as the first base to any one of the 24^{th} to 44^{th} bases in a direction toward the 5' end in the base sequence of SEQ ID NO: 2, with the cytosine base (c) being the 3' end, and
(R): oligonucleotide complementary to a region extending from a cytosine base (c) at position 255 to be considered as the first base to any one of the 23^{rd} to 47^{th} bases in a direction toward the 3' end in the base sequence of SEQ ID NO: 2, with a guanine base (g) complementary to the cytosine base (c) at position 255 being the 3' end.

10. The method according to claim 9, wherein the oligonucleotide (F) is the following oligonucleotide (F1) and the oligonucleotide (R) is the following oligonucleotide (R1):
(F1) oligonucleotide consisting of the base sequence of SEQ ID NO: 25, and
(R1) oligonucleotide consisting of the base sequence of SEQ ID NO: 51.

11. The method according to claim 5, wherein the labeled probe is labeled with a fluorescent label, and fluorescence intensity of the fluorescent label is measured in the process (B) or
wherein the nucleic acid to be tested is nucleic acid derived from a biological sample.

12. The method according to claim 11, wherein the biological sample is blood.

13. A mutation detection kit that is used for a method of detecting a mutation in the JAK2 gene according to claim 5, wherein the mutation detection kit comprises a probe according to claim 1, optionally
further comprising a primer set for amplifying the JAK2 gene by a nucleic acid amplification technique,
wherein the primer set comprises a pair of primers including a forward primer composed of the following oligonucleotide (F) and a reverse primer composed of the following oligonucleotide (R):
(F): oligonucleotide having a sequence identical to that of a region extending from a cytosine base (c) at position 193 to be considered as the first base to any one of the 24th to 44th bases in a direction toward the 5' end in the base sequence of SEQ ID NO: 2, with the cytosine base (c) being the 3' end, and
(R): oligonucleotide complementary to a region extending from a cytosine base (c) at position 255 to be considered as the first base to any one of the 23rd to 47th bases in a direction toward the 3' end in the base sequence of SEQ ID NO: 2, with a guanine base (g) complementary to the cytosine base (c) at position 255 being the 3' end.

14. A mutation detection kit as claimed in claim 13 wherein the oligonucleotide (F) is the following oligonucleotide (F1) and the oligonucleotide (R) is the following oligonucleotide (R1):
(F1) oligonucleotide consisting of the base sequence of SEQ ID NO: 25, and
(R1) oligonucleotide consisting of the base sequence of SEQ ID NO: 51, or
wherein the primer set is one for amplifying the JAK2 gene derived from a biological sample.

## Patentansprüche

1. Eine Sonde zum Nachweisen einer Mutation im JAK2-Gen, wobei die Sonde aus Oligonukleotid (X) besteht, das eine Sequenz aufweist, die identisch zu der eines Bereichs ist, der sich von einer Cytosinbase (c) an Position 84, die als die erste Base betrachtet wird, zu einer der 17. bis 22. Base in einer Richtung zu dem 5'-Ende in Exon 12 des JAK2-Gens erstreckt, das aus der Basensequenz der SEQ ID NR: 1 besteht, wobei die Cytosinbase (c) das 3'-Ende ist, wobei die Cytosinbase an dem 3'-Ende mit einer Markierungssubstanz markiert ist.

2. Die Sonde gemäß Anspruch 1, wobei Oligonukleotid (X) ein Oligonukleotid ist, das aus einer Basensequenz, ausgewählt aus der Gruppe bestehend aus SEQ ID NR: 3 bis 8 besteht, oder wobei eine Base (k) in der Basensequenz der SEQ ID NR: 3 bis 8 Thymin (t) ist, oder
wobei die Sonde eine zum Nachweisen einer Mutation (g → t) einer Base an Position 73 in der Basensequenz der SEQ ID NR: 1 ist.

3. Die Sonde gemäß Anspruch 1, wobei die markierte Sonde eine ist, die unabhängig ein Signal aufweist, jedoch kein Signal nach der Hybridisierung aufweist oder eine ist, die unabhängig kein Signal aufweist, jedoch ein Signal nach der Hybridisierung aufweist, oder wobei die markierte Sonde eine Sonde ist, die mit einem fluoreszierenden Farbstoff markiert ist und eine Sonde ist, die unabhängig Fluoreszenz aufweist und es der Fluoreszenz ermöglicht, nach der Hybridisierung abzunehmen.

4. Die Sonde gemäß Anspruch 1, wobei die Sonde eine Sonde zur Schmelztemperaturanalyse (Tm) ist.

5. Ein Verfahren zum Nachweisen einer Mutation im JAK2-Gen, wobei das Verfahren die nachstehenden Prozesse (A) bis (C) umfasst:
(A) Bereitstellen eines Reaktionssystems, das eine Sonde gemäß Anspruch 1 und eine zu testende Nukleinsäure einschließt, zum Nachweisen des Vorliegens oder der Abwesenheit einer Mutation,
(B) Messen von Signalwerten, die Schmelzzustände eines Hybridisierungsprodukts zwischen der Probe und der zu testenden Nukleinsäure anzeigen, während die Temperatur des Reaktionssystems verändert wird, und
(C) Bestimmen des Vorliegens oder der Abwesenheit der Mutation in der zu testenden Nukleinsäure aus einer Veränderung in den Signalwerten, die eine Veränderung in der Temperatur begleiten.

6. Das Verfahren gemäß Anspruch 5, wobei die Mutation in dem JAK2-Gen eine Mutation
(g → t) einer Base an Position 73 in Exon 12 des JAK2-Gens ist, das in SEQ ID NR: 1 angezeigt ist, ist.

7. Das Verfahren gemäß Anspruch 5, wobei die zu testende Nukleinsäure, die in dem Prozess (A) verwendet wird ein Amplifikationsprodukt ist, das von einer Templatenukleinsäure durch eine Nukleinsäureamplifikationstechnik amplifiziert wurde.

8. Das Verfahren gemäß Anspruch 7, wobei der Prozess (A) ferner einen Prozess der Amplifikation von zu testender Nukleinsäure von Templatenukleinsäure unter Verwenden eines Primersets umfasst.

9. Das Verfahren gemäß Anspruch 8, wobei das Primerset ein Primerpaar umfasst, das einen Forward-Primer, der aus dem nachstehenden Oligonukleotid (F) aufgebaut ist und einen Reverse-Primer, der aus dem nachstehenden Oligonukleotid (R) aufgebaut ist, umfasst:
(F): Oligonukleotid mit einer Sequenz, die identisch ist zu der eines Bereichs, der sich von einer Cytosinbase (c) an Position 193, die als die erste Base betrachtet wird, zu einer der 24. bis 44. Base, in eine Richtung zu dem 5'-Ende in der Basensequenz von SEQ ID NR: 2 erstreckt, wobei die Cytosinbase (c) das 3'-Ende ist, und
(R): Oligonukleotid, das komplementär zu einem Bereich ist, der sich von einer Cytosinbase (c) an Position 255, die als die erste Base betrachtet wird, zu einer der 23. bis 47. Base, in eine Richtung zu dem 3'-Ende in der Basensequenz von SEQ ID NR: 2 erstreckt, mit einer Guaninbase (g), die komplementär zu der Cytosinbase (c) an Position 255, die das 3'-Ende ist, vorliegt.

10. Das Verfahren gemäß Anspruch 9, wobei das Oligonukleotid (F) das nachstehende Oligonukleotid (F1) ist und das Oligonukleotid (R) das nachstehende Oligonukleotid (R1) ist:
(F1) Oligonukleotid bestehend aus der Basensequenz von SEQ ID NR: 25, und
(R1) Oligonukleotid bestehend aus der Basensequenz von SEQ ID NR: 51.

11. Das Verfahren gemäß Anspruch 5, wobei die markierte Sonde mit einem fluoreszierenden Markierungsmittel markiert ist und die Fluoreszenzintensität des fluoreszierenden Markierungsmittel in dem Prozess (B) gemessen wird oder
wobei die zu testende Nukleinsäure Nukleinsäure ist, die sich von einer biologischen Probe ableitet.

12. Das Verfahren gemäß Anspruch 11, wobei die biologische Probe Blut ist.

13. Ein Mutationsnachweiskit, das für ein Verfahren zum Nachweisen einer Mutation in dem JAK2-Gen gemäß Anspruch 5 verwendet wird, wobei das Mutationsnachweiskit eine Sonde gemäß Anspruch 1 umfasst, gegebenenfalls
ferner ein Primerset zur Amplifikation des JAK2-Gens durch eine Nukleinsäureamplifikationstechnik umfassend,
wobei das Primerset ein Primerpaar umfasst, die einen Forward-Primer, das aus dem nachstehenden Oligonukleotid (F) aufgebaut ist und einen Reverse-Primer, der aus dem nachstehenden Oligonukleotid (R) aufgebaut ist, umfasst:
(F): Oligonukleotid mit einer Sequenz, die identisch ist zu der eines Bereichs, der sich von einer Cytosinbase (c) an Position 193, die als die erste Base betrachtet wird, zu einer der 24. bis 44. Base, in eine Richtung zu dem 5'-Ende in der Basensequenz von SEQ ID NR: 2 erstreckt, wobei die Cytosinbase (c) das 3'-Ende ist, und
(R): Oligonukleotid, das komplementär zu einem Bereich ist, der sich von einer Cytosinbase (c) an Position 255, die als die erste Base betrachtet wird, zu einer der 23. bis 47. Base, in eine Richtung zu dem 3'-Ende in der Basensequenz von SEQ ID NR: 2 erstreckt, mit einer Guaninbase (g), die komplementär zu der Cytosinbase (c) an Position 255, die das 3'-Ende ist, vorliegt.

14. Ein Mutationsnachweiskit wie in Anspruch 13 beansprucht, wobei das Oligonukleotid (F) das nachstehende Oligonukleotid (F1) ist und das Oligonukleotid (R) das nachstehende Oligonukleotid (R1) ist:
(F1) Oligonukleotid bestehend aus der Basensequenz der SEQ ID NR: 25, und
(R1) Oligonukleotid bestehend aus der Basensequenz von SEQ ID NR: 51, oder
wobei das Primerset eines zum Amplifizieren des JAK2-Gens ist, das sich von einer biologischen Probe ableitet.

## Revendications

1. Sonde de détection d'une mutation dans le gène JAK2,
dans laquelle la sonde est constituée d'un oligonucléotide (X) ayant une séquence identique à celle d'une région s'étendant d'une base de cytosine (c) en position 84, à considérer comme la première base, à l'une quelconque des 17^{ème} à 22^{ème} bases dans le sens vers la terminaison 5' dans l'exon 12 du gène JAK2 constitué de la séquence de bases SEQ ID n° 1, avec la base de cytosine (c) qui est la terminaison 3', dans laquelle la base de cytosine à la terminaison 3' est marquée par une substance de marquage.

2. Sonde selon la revendication 1, dans laquelle l'oligonucléotide (X) est un oligonucléotide constitué d'une séquence de bases choisie dans le groupe constitué des SEQ ID n° 3 à 8, ou
dans laquelle une base (k) de la séquence de bases des SEQ ID n° 3 à 8 est la thymine (t), ou
dans laquelle la sonde est une sonde de détection d'une mutation (g→t) d'une base en position 73 dans la séquence de bases SEQ ID n° 1.

3. Sonde selon la revendication 1, dans laquelle la sonde marquée est une sonde qui présente un signal indépendamment, mais ne présente pas un signal après hybridation, ou une sonde qui ne présente pas un signal indépendamment, mais présente un signal après hybridation, ou
dans laquelle la sonde marquée est une sonde marquée par un colorant fluorescent et est une sonde qui présente une fluorescence indépendamment et permet à la fluorescence de diminuer après hybridation.

4. Sonde selon la revendication 1, dans laquelle la sonde est une sonde pour l'analyse de la température de fusion (Tm).

5. Procédé de détection d'une mutation dans le gène JAK2,
dans lequel le procédé comprend les processus suivants (A) à (C) consistant à :
(A) mettre en oeuvre un système réactionnel comprenant une sonde selon la revendication 1 et un acide nucléique à tester pour détecter la présence ou l'absence de la mutation,
(B) mesurer des valeurs de signal qui indiquent les états de fusion d'un produit d'hybridation entre la sonde et l'acide nucléique à tester tout en changeant la température du système réactionnel, et
(C) déterminer la présence ou l'absence de la mutation dans l'acide nucléique à tester, à partir d'un changement des valeurs de signal accompagnant un changement de la température.

6. Procédé selon la revendication 5, dans lequel la mutation dans le gène JAK2 est une mutation (g→t) d'une base en position 73 dans l'exon 12 du gène JAK2 indiqué dans la SEQ ID n° 1.

7. Procédé selon la revendication 5, dans lequel l'acide nucléique à tester qui est utilisé dans le processus (A) est un produit d'amplification qui a été amplifié à partir d'acide nucléique matrice par une technique d'amplification d'acide nucléique.

8. Procédé selon la revendication 7, dans lequel le processus (A) comprend en outre un procédé d'amplification de l'acide nucléique à tester à partir d'un acide nucléique matrice en utilisant un ensemble d'amorces.

9. Procédé selon la revendication 8, dans lequel l'ensemble d'amorces comprend une paire d'amorces comprenant une amorce sens composée de l'oligonucléotide suivant (F) et une amorce antisens composée de l'oligonucléotide suivant (R) :
(F) : oligonucléotide ayant une séquence identique à celle d'une région s'étendant d'une base de cytosine (c) en position 193, à considérer comme la première base, à l'une quelconque des 24^{ème} à 44^{ème} bases dans le sens vers la terminaison 5' dans la séquence de bases SEQ ID n° 2, avec la base de cytosine (c) qui est la terminaison 3', et
(R) : oligonucléotide complémentaire d'une région s'étendant d'une base de cytosine (c) en position 255, à considérer comme la première base, à l'une quelconque des 23^{ème} à 47^{ème} bases dans le sens vers la terminaison 3' dans la séquence de bases SEQ ID n° 2, avec une base de guanine (g) complémentaire de la base de cytosine (c) en position 255 qui est la terminaison 3'.

10. Procédé selon la revendication 9, dans lequel l'oligonucléotide (F) est l'oligonucléotide suivant (F1) et l'oligonucléotide (R) est l'oligonucléotide suivant (R1) :
(F1) : oligonucléotide constitué de la séquence de bases SEQ ID n° 25 et
(R1) : oligonucléotide constitué de la séquence de bases SEQ ID n° 51.

11. Procédé selon la revendication 5, dans lequel la sonde marquée est marquée par un marqueur fluorescent et l'intensité de la fluorescence du marqueur fluorescent est mesurée dans le processus (B), ou
dans lequel l'acide nucléique à tester est de l'acide nucléique tiré d'un échantillon biologique.

12. Procédé selon la revendication 11, dans lequel l'échantillon biologique est le sang.

13. Kit de détection de mutation qui est utilisé pour un procédé de détection d'une mutation dans le gène JAK2 selon la revendication 5, dans lequel le kit de détection de mutation comprend une sonde selon la revendication 1, éventuellement
comprenant en outre un ensemble d'amorces pour amplifier le gène JAK2 par une technique d'amplification d'acide nucléique,
dans lequel l'ensemble d'amorces comprend une paire d'amorces comprenant une amorce sens composée de l'oligonucléotide suivant (F) et une amorce antisens composée de l'oligonucléotide suivant (R) :
(F) : oligonucléotide ayant une séquence identique à celle d'une région s'étendant d'une base de cytosine (c) en position 193, à considérer comme la première base, à l'une quelconque des 24^{ème} à 44^{ème} bases dans le sens vers la terminaison 5' dans la séquence de bases SEQ ID n° 2, avec la base de cytosine (c) qui est la terminaison 3', et
(R) : oligonucléotide complémentaire d'une région s'étendant d'une base de cytosine (c) en position 255, à considérer comme la première base, à l'une quelconque des 23^{ème} à 47^{ème} bases dans le sens vers la terminaison 3' dans la séquence de bases SEQ ID n° 2, avec une base de guanine (g) complémentaire de la base de cytosine (c) en position 255 qui est la terminaison 3'.

14. Kit de détection de mutation selon la revendication 13, dans lequel l'oligonucléotide (F) est l'oligonucléotide suivant (F1) et l'oligonucléotide (R) est l'oligonucléotide suivant (R1) :
(F1) : oligonucléotide constitué de la séquence de bases SEQ ID n° 25 et
(R1) : oligonucléotide constitué de la séquence de bases SEQ ID n° 51, ou
dans lequel l'ensemble d'amorces est un ensemble pour amplifier le gène JAK2 tiré d'un échantillon biologique.


### SEQUENCE LISTING

<110> ARKRAY Inc.
<120> TF08020-01
<130> Pr obe t o det ect JAK2 gene mutation and use the same
<150> JP 07/184842
   <151> 2007-07-13
<160> 55
<170> Patent In version 3. 1
<210> 1
   <211> 88
   <212> DNA
   <213> Horm sapi ens
<220>
   <221> misc_feature
   <222> (73)..(73)
   <223> k st ands for g or t
<400> 1
   tctttctttg aagcagcaag tatgatgagc aagctttctc acaagcattt ggttttaaat    60
   t at ggagt at gt kt ct gt gg agacgaga 88
<210> 2
   <211> 500
   <212> DNA
   <213> Homo sapiens
<220>
   <221> exon
   <222> (154)..(241)
   <223>
<220>
   <221> misc_feature
   <222> (226)..(226)
   <223> k stands for g or t
<400> 2
   gtccatataa agggaccaaa gcacattgta tcctcatcta tagtcatgct gaaagtagga     60
   gaaagtgcat ctttattatg gcagagagaa ttttctgaac tatttatgga caacagtcaa    120
   acaacaattc tttgtacttt tttttttcct tag tct ttc ttt gaa gca gca agt     174
                                        Ser Phe Phe Glu Ala Ala Ser
                                        1               5
   atg atg agc aag ctt tct cac aag cat ttg gtt tta aat tat gga gta      222
   Met Met Ser Lys Leu Ser His Lys His Leu Val Leu Asn Tyr Gly Val
           10                  15                  20
   tgt ktc tgt gga gac gag a gtaagtaaaa ctacaggctt tctaatgcct           271
   Cys Xaa Cys Gly Asp Glu
       25
   ttctcagagc atctgttttt gtttatatag aaaattcagt ttcaggatca cagctaggtg    331
   tcagtgtaaa ctataattta acaggagtta agtatttttg aaactgaaaa cactgtagga    391
   ctattcagtt atatcttgtg aaaaaggaaa gcaatgaagt taaaagtaga aggttacaat    451
   gcccaaacaa tagagtatta tagtaaacaa atgtctataa aacattttg               500
<210> 3
   <211> 17
   <212> DNA
   <213> Artificial
<220>
   <223> pr obe t o det ect JAK2 gene mutation
<220>
   <221> misc_feat ur e
   <222> (6)..(6)
   <223> k st ands f or g or t
<400> 3
   t at gt kt ct g t ggagac 17
<210> 4
   <211> 18
   <212> DNA
   <213> Artificial
<220>
   <223> pr obe to detect JAK2 gene mutation
<220>
   <221> misc_feature
   <222> (7)..(7)
   <223> k st ands f or g or t
<400> 4
   gt at gt kt ct gt ggagac 18
<210> 5
   <211> 19
   <212> DNA
   <213> Artificial
<220>
   <223> pr obe t o det ect JAK2 gene mutation
<220>
   <221> misc_feature
   <222> (8)..(8)
   <223> k stands for g or t
<400> 5
   agt at gt kt c t gt ggagac 19
<210> 6
   <211> 20
   <212> DNA
   <213> Artificial
<220>
   <223> pr obe t o det ect JAK2 gene mutation
<220>
   <221> misc_feature
   <222> (9)..(9)
   <223> k st ands f or g or t
<400> 6
   gagt at gt kt ct gt ggagac 20
<210> 7
   <211> 21
   <212> DNA
   <213> Artificial
<220>
   <223> pr obe to detect JAK2 gene mitation
<220>
   <221> misc_feat ur e
   <222> (10)..(10)
   <223> k st ands f or g or t
<400> 7
   ggagt at gt k t ct gt ggaga c 21
<210> 8
   <211> 22
   <212> DNA
   <213> Artificial
<220>
   <223> probe t o det ect JAK2 gene mutation
<220>
   <221> misc_f eat ur e
   <222> (11)..(11)
   <223> k stands for g or t
<400> 8
   t ggagt at gt kt ct gt ggag ac 22
<210> 9
   <211> 44
   <212> DNA
   <213> Artificial
<220>
   <223> forward primer
<400> 9
   t t agt ct t t c t t t gaagcag caagt at gat gagcaagct t t ct c 44
<210> 10
   <211> 43
   <212> DNA
   <213> Artificial
<220>
   <223> forward primer
<400> 10
   t agt ct t t ct t t gaagcagc aagt at gat g agcaagct t t ct c 43
<210> 11
   <211> 42
   <212> DNA
   <213> Artificial
<220>
   <223> forward primer
<400> 11
   agt ct t t ct t t gaagcagca agt at gat ga gcaagct t t c t c 42
<210> 12
   <211> 41
   <212> DNA
   <213> Artificial
<220>
   <223> f or war d primer
<400> 12
   gt ct t t ct t t gaagcagcaa gt at gat gag caagct t t ct c 41
<210> 13
   <211> 40
   <212> DNA
   <213> Artificial
<220>
   <223> forward primer
<400> 13
   t ct t t ct t t g aagcagcaag t at gat gagc aagct t t ct c 40
<210> 14
   <211> 39
   <212> DNA
   <213> Artificial
<220>
   <223> f or war d primer
<400> 14
   ct t t ct t t ga agcagcaagt at gat gagca agct t t ct c 39
<210> 15
   <211> 38
   <212> DNA
   <213> Artificial
<220>
   <223> f or war d primer
<400> 15
   t t t ct t t gaa gcagcaagt a t gat gagcaa gct t t ct c 38
<210> 16
   <211> 37
   <212> DNA
   <213> Artificial
<220>
   <223> f or war d primer
<400> 16
   t t ct t t gaag cagcaagt at gat gagcaag ct t t ct c 37
<210> 17
   <211> 36
   <212> DNA
   <213> Artificial
<220>
   <223> f or war d primer
<400> 17
   t ct t t gaagc agcaagt at g at gagcaagc t t t ct c 36
<210> 18
   <211> 35
   <212> DNA
   <213> Artificial
<220>
   <223> f or war d primer
<400> 18
   ct t t gaagca gcaagt at ga t gagcaagct t t ct c 35
<210> 19
   <211> 34
   <212> DNA
   <213> Artificial
<220>
   <223> forward primer
<400> 19
   t t t gaagcag caagt at gat gagcaagct t t ct c 34
<210> 20
   <211> 33
   <212> DNA
   <213> Artificial
<220>
   <223> f or war d primer
<400> 20
   t t gaagcagc aagt at gat g agcaagcttt t ct c 33
<210> 21
   <211> 32
   <212> DNA
   <213> Artificial
<220>
   <223> f or war d primer
<400> 21
   t gaagcagca agt at gat ga gcaagct t t c t c 32
<210> 22
   <211> 31
   <212> DNA
   <213> Artificial
<220>
   <223> f or war d primer
<400> 22
   gaagcagcaa gt at gat gag caagct t t ct c 31
<210> 23
   <211> 30
   <212> DNA
   <213> Artificial
<220>
   <223> f or war d primer
<400> 23
   aagcagcaag t at gat gagc aagct t t ct c 30
<210> 24
   <211> 29
   <212> DNA
   <213> Artificial
<220>
   <223> f or war d primer
<400> 24
   agcagcaagt at gat gagca agct t t ct c 29
<210> 25
   <211> 28
   <212> DNA
   <213> Artificial
<220>
   <223> f or war d primer
<400> 25
   gcagcaagta tgatgagcaa gct t t ct c 28
<210> 26
   <211> 27
   <212> DNA
   <213> Artificial
<220>
   <223> forward primer
<400> 26
   cagcaagtat gat gagcaag ct t t ct c 27
<210> 27
   <211> 26
   <212> DNA
   <213> Artificial
<220>
   <223> forward primer
<400> 27
   agcaagt at g at gagcaagc t t t ct c 26
<210> 28
   <211> 25
   <212> DNA
   <213> Artificial
<220>
   <223> f or ward primer
<400> 28
   gcaagt at ga t gagcaagct t t ct c 25
<210> 29
   <211> 24
   <212> DNA
   <213> Artificial
<220>
   <223> f or war d primer
<400> 29
   caagt at gat gagcaagct t t ct c 24
<210> 30
   <211> 23
   <212> DNA
   <213> Artificial
<220>
   <223> f or war d primer
<400> 30
   aagt at gat g agcaagct t t ct c 23
<210> 31
   <211> 47
   <212> DNA
   <213> Artificial
<220>
   <223> reverse primer
<400> 31
   ct at at aaac aaaaacagat gct ct gagaa aggcat t aga aagcct g 47
<210> 32
   <211> 46
   <212> DNA
   <213> Artificial
<220>
   <223> r ever se primer
<400> 32
   t at at aaaca aaaacagat g ct ct gagaaa ggcat t agaa agcct g 46
<210> 33
   <211> 45
   <212> DNA
   <213> Artificial
<220>
   <223> r ever se primer
<400> 33
   at at aaacaa aaacagat gc t ct gagaaag gcat t agaaa gcct g 45
<210> 34
   <211> 44
   <212> DNA
   <213> Artificial
<220>
   <223> reverse primer
<400> 34
   t at aaacaaa aacagat gct ct gagaaagg cat t agaaag cct g 44
<210> 35
   <211> 43
   <212> DNA
   <213> Artificial
<220>
   <223> r ever se primer
<400> 35
   at aaacaaaa acagat gct c t gagaaaggc at t agaaagc ct g 43
<210> 36
   <211> 42
   <212> DNA
   <213> Artificial
<220>
   <223> reverse primer
<400> 36
   t aaacaaaaa cagat gct ct gagaaaggca t t agaaagcc t g 42
<210> 37
   <211> 41
   <212> DNA
   <213> Artificial
<220>
   <223> reverse primer
<400> 37
   aaacaaaaac agat gct ct g agaaaggcat t agaaagcct g 41
<210> 38
   <211> 40
   <212> DNA
   <213> Artificial
<220>
   <223> reverse primer
<400> 38
   aacaaaaaca gat gct ct ga gaaaggcat t agaaagcct g 40
<210> 39
   <211> 39
   <212> DNA
   <213> Artificial
<220>
   <223> r ever se primer
<400> 39
   acaaaaacag at gct ct gag aaaggcatta gaaagcctg 39
<210> 40
   <211> 38
   <212> DNA
   <213> Artificial
<220>
   <223> r ever se primer
<400> 40
   caaaaacaga t gct ct gaga aaggcat t ag aaagcct g 38
<210> 41
   <211> 37
   <212> DNA
   <213> Artificial
<220>
   <223> reverse primer
<400> 41
   aaaaacagat gct ct gagaa aggcattaga aagcct g 37
<210> 42
   <211> 36
   <212> DNA
   <213> Artificial
<220>
   <223> reverse primer
<400> 42
   aaaacagat g ct ct gagaaa ggcat t agaa agcct g 36
<210> 43
   <211> 35
   <212> DNA
   <213> Artificial
<220>
   <223> reverse primer
<400> 43
   aaacagatgc t ct gagaaag gcattagaaa gcctg 35
<210> 44
   <211> 34
   <212> DNA
   <213> Artificial
<220>
   <223> r ever se primer
<400> 44
   aacagatgct ctgagaaagg cattagaaag cctg 34
<210> 45
   <211> 33
   <212> DNA
   <213> Artificial
<220>
   <223> reverse primer
<400> 45
   acagatgctc c t gagaaaggc attagaaagc ctg 33
<210> 46
   <211> 32
   <212> DNA
   <213> Artificial
<220>
   <223> r ever se primer
<400> 46
   cagat gct ct gagaaaggca t t agaaagcc t g 32
<210> 47
   <211> 31
   <212> DNA
   <213> Artificial
<220>
   <223> r ever se primer
<400> 47
   agat gct ct g agaaaggcat t agaaagcct g 31
<210> 48
   <211> 30
   <212> DNA
   <213> Artificial
<220>
   <223> r ever se primer
<400> 48
   gatgctctga gaaaggcat t agaaagcct g 30
<210> 49
   <211> 29
   <212> DNA
   <213> Artificial
<220>
   <223> r ever se primer
<400> 49
   at gct ct gag aaaggcat t a gaaagcct g 29
<210> 50
   <211> 28
   <212> DNA
   <213> Artificial
<220>
   <223> r ever se primer
<400> 50
   t gct ct gaga aaggcat t ag aaagcct g 28
<210> 51
   <211> 27
   <212> DNA
   <213> Artificial
<220>
   <223> reverse pri rrer
<400> 51
   gctctgagaa aggcattaga aagcctg 27
<210> 52
   <211> 26
   <212> DNA
   <213> Artificial
<220>
   <223> reverse primer
<400> 52
   ctctgagaaa ggcattagaa agcctg 26
<210> 53
   <211> 25
   <212> DNA
   <213> Artificial
<220>
   <223> r ever se primer
<400> 53
   t ct gagaaag gcat t agaaa gcct g 25
<210> 54
   <211> 24
   <212> DNA
   <213> Artificial
<220>
   <223> reverse primer
<400> 54
   ct gagaaagg cat t agaaag cct g 24
<210> 55
   <211> 23
   <212> DNA
   <213> Artificial
<220>
   <223> reverse primer
<400> 55
   t gagaaaggc at t agaaagc ct g 23